# EUROPÄISCHE PATENTSCHRIFT

(11) **EP 1 617 913 B1**
(45) Veröffentlichungstag und Bekanntmachung des Hinweises auf die Patenterteilung: **18.03.2009**
(21) Anmeldenummer: 04726108.6
(22) Anmeldetag: 07.04.2004
(51) Int. Cl.: A61Q 15/00, A61Q 17/04, A61Q 19/00, A61K 8/31

(54) **POLY-ALPHA-OLEFIN-HALTIGE KOSMETISCHE ZUSAMMENSETZUNG**
POLY-ALPHA-OLEFIN-CONTAINING COSMETIC COMPOSITION
COMPOSITION COSMETIQUE CONTENANT UNE POLY-ALPHA-OLEFINE

(30) Priorität: 16.04.2003 DE 10317781
(43) Veröffentlichungstag der Anmeldung: 25.01.2006
(73) Patentinhaber: Cognis IP Management GmbH, 40589 Düsseldorf (DE)
(72) Erfinder: DIERKER, Markus, 40597 Düsseldorf (DE); PRINZ, Daniela, 41542 Dormagen (DE); WESTFECHTEL, Alfred, 40724 Hilden (DE); ZANDER, Lars, 41569 Rommerkirchen (US); ANSMANN, Achim, 40699 Erkrath (DE)
(86) Internationale Anmeldenummer: PCT/EP2004/003699
(87) Internationale Veröffentlichungsnummer: WO 2004/091565

(56) Entgegenhaltungen:
- EP-A- 0 547 897
- EP-A- 0 804 921
- WO-A-99/62468
- US-B1- 6 464 967

## Beschreibung

### Gebiet der Erfindung

Die Erfindung betrifft neuartige kosmetische Zusammensetzungen, die bestimmte Poly-α-Olefine enthalten, sowie die Verwendung dieser Poly-α-Olefine als Ölkörper in kosmetischen und pharmazeuti-schen Zubereltungen. Die kosmetischen oder pharmazeutischen Zubereitungen welsen eine gute dermatologische Verträglichkelt auf und vermitteln ein besonders leichtes Hautgefühl.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erschelnungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkelt zur erfüllen, werden kontinuierlich neue Ölkörper und Emulgator-Gemische entwickelt und getestet. Zur Herstellung kosmetischer bzw, pharmazeutischer Zubereitungen werden eine Vielzahl von natürlichen und synthetischen Ölen, beispielsweise Mandel- oder Avocadoöl, Esteröle, Ether, Alkylcarbonate, Kohlenwasserstoffe sowie Silikonöle eingesetzt. Eine wesentliche Aufgabe der Ölkomponenten ist es, neben der pflegenden Wirkung, die in unmittelbarem Zusammenhang mit der Haulfettung steht, dem Konsumenten ein nichtklebriges, möglich rasch eintretendes und lang anhaltendes Gefühl der Hautglätte und geschmeidigkeit zu vermitteln.

EP-A-0 547 897, EP-A-0 804 921, US-B-6 464 967, sowie WO 99/62 468 beschreiben kosmetische Zubereitungen, welche Poly-α-Olefine enthalten.

Das subjektive Empfinden auf der Haut kann mit dem physikochomischen Parameter der Spreitung der Ölkörper auf der Haut korreliert und objektiviert werden, wie dies von U. Zeldler in der Fachzeitschrift Fette, Seifen, Anstrichmittel 87, 403 (1986) dargestellt wurde. Demnach lassen sich kosmetische Ölkörper In niedrispreitende (< 300 mm² / 10 min), mittelspreitende (>/=300 bis <1000 mm² / 10 min.) und hochspreitende Öle (>/= 1000 mm² / 10 min) eintellen. Setzt man in einer vorgegebenen Formulierung als Ölkörper ein hochspreitendes Öl ein, so wird zwar sehr rasch das gewünschte Gefühl der Hautglättung erzielt, und es wird im Falle der Verwendung von Cyclomethiconen, z.B Dow Corning 245 fluid (Dow Corning Corporation) oder Abil^{®} B 8839 (Goldschmidt Chemical Coperation) zusätzlich ein vom Verbraucher erwünschtes samtiges Endgefühl gefunden. Dieses Erlebnis dauert jedoch nicht lange an, da durch die hohe Flüchtigkeit der zuletzt genannten Strukturen das ausgeprägte Glättegefühl und damit die Samtigkelt sehr rasch verschwindet und ein unangenehmes stumpfes Gefühl auf der Haut zurück bleibt.

Cyclomethicone haben jedoch gegenüber anderen Emollients auf Kohlenwasserstoffbasis wie sehr leichten Mineralölen, Polybutylenen (z. B. Arlamol^{®} HD, ICI), Ethylhexylcylohexan (Cetlol^{®} S, Cognis Deutschland GmbH & Co. KG) den Vorteil, dass sie auf der Haut als sehr leicht empfunden werden. Es bestand daher ein Bedarf an Ölkörpern/Emollients auf Kohlenwasserstoffbasis, die die Vorteile der Cyclomethicone wie leichtes Hautgefühl und gute Spreiteigenschaften auf sich vereinigen, ohne die Nachtelle der Cyclomethicone zu zeigen.

Aufgabe der vorliegenden Erfindung ist es, verbesserte, hochspreitende Ölkörper und diese enthaltende Zubereitungen zur Verfügung zu stellen, die ein rasch eintretendes und länger anhaltendes Gefühl der Heutglätte vermitteln und über eine gute Hautverträglichkeit verfügen. Darüber hinaus soll der Ölkörper einfach und stabil In Emulsionen einzuarbelten und hydrolyseunempfindlich gegenüber pH - Schwankungen sein und zu niedrigviskosen Zusammensetzungen führen, die ein sehr leichtes Hautgefühl vermitteln.

### Beschreibung der Erfindung

Die Erfindung betrifft eine kosmetische Zusammensetzung in Form von Emulsionen oder Dispersionen, die Wasser und Ölphase nebeneinander enthalten, die wenigstens ein Poly-α-Olefin enthält, welches dadurch erhältlich ist, dass man wenigstens einen primären Alkohol in Gegenwart sauren Aluminoschichtsilikats einer dehydratisierenden Polymerisation bei einer Temperatur im Bereich von 60 bis 340 °C unterwirft, wobei der primäre Alkohol ausgewählt ist aus der Gruppe
a) ungesittigter monofunktioneller Alkohole,
b) verzweigter monofunktioneller Alkohole und
c) difunktioneller Alkohole.

Die Poly-α-Olefine, die in der erfindungemäßen kosmetischen Zusammensetzung zum Einsatz kommen, sind an sich bereits beschrieben worden. Sie werden in der deutschen Patentanmeldung DE 10152267 der Anmelderin sowie deren internationaler Patentanmeldung PCT/EP02/11392 erwähnt. Neben der Beschreibung der Verbindungen selbst finden sich dort auch detaillierte Informationen zu ihrer Herstellung. Auf die entsprechenden Anmeldungen wird hier Bezug genommen.

Nur kurz zusammenfassend sei erwähnt, dass sie Umsetzung des primären Alkohols bevorzugt unter Schutzgas unter kontinuierlicher Abscheidung des entstehenden Wassers erfolgt. Das als Katalysator eingesetzte saure Aluminoschichtsilikat hat bevorzugt eine Säurebeladung von 3 bis 300 mval/100g. Beispiele für Aluminoschichtsilikate sind Talk sowie Tone mit Blattstruktur wie Kaolinit, Montmorillonit, Bentonite und Hectorite. Sinnvoll ist es, die Umsetzung unter Wasserabscheidung solange durchzuführen, bis keine weitere Abspaltung von Wasser mehr erfolgt. Die Reaktionszeiten liegen üblicherweise im Bereich von 2 bis 48 Stunden. Anschließend wird der Katalysator beispielsweise durch Filtration entfernt. Der Oligomerisierungsgrad der Poly-α-Olefine liegt im Bereich von 1 bis 10. Eine gezielte Einstellung des Oligomerisierungsgrades kann dadurch erreicht werden, dass man das bei der kontinuierlichen Wasserabscheidung mitgeschleppte Olefin wieder in die Reaktionsmischung zurückführt; was zu höheren Oligomerisierungsgraden führt. Die erhaltenen Poly-α-Olefine sind geruchlose, farblose oder gelbliche Produkte, die flüssig oder fest sein können.
Eine genaue Strukturformel für die erhaltenen Poly-α-Olefine kann nicht angegeben werden, da die fraglichen primären Alkohole unten den dehydratisierenden Bedingungen bei der Polymerisation in verschiedenste ungesättigte Monomere isomerisiert werden, die dann miteinander polymerisieren.

Die erwähnten primären Alkohole können einzeln oder im Gemisch untereinander eingesetzt werden. Während der Alkylrest der Alkohole der Gruppe b) verzweigt ist, können die Alkylreste der primären Alkohole der Gruppen a) und c) entweder geradkettig oder verzweigt sein. Die ungesättigten Alkohole können einfach oder mehrfach ungesättigt sein und sind insbesondere olefinisch ungesättigt.

Bevorzugte kosmetische Zusammensetzungen sind solche, in denen der primäre Alkohol 6 bis 72 Kohlenstoffatome und insbesondere 6 bis 24 Kohlenstoffatome aufweist.

Als Alkohol der Gruppe a) wird bevorzugt ein linearer Alkohol eingesetzt. Beispiele ungesättigter monofunktioneller Alkohole der Gruppe a) sind 10-Undecen-1-ol, Oleylalkohol, Elaidylalkohol, Ricinolalkohol, Linoleylalkohol, Linolenylalkohol, Gadoleylalkohol, Erucaalkohol und Brassidylalkohol.

Als Alkohol der Gruppe b) wird vorzugsweise ein Alkohol eingesetzt, der ausgewählt ist aus der Gruppe der verzweigten Alkohole mit b1) mindestens einer Methylgruppe und insbesondere 1 bis 6 Methylverzweigungen im Alkylrest, b2) einer C₂-C₁₈-Verzweigung im Alkylrest und b3) einer C₂-C₁₈-Verzweigung in α-Stellung zur endständigen CH₂OH-Gruppe.
Im Fall der Gruppe b1) mit mindestens einer Methylverzweigung in der Alkylgruppe kann der Methylrest an jeder beliebigen Stelle in der Alkylkette positioniert sein. Geeignete Beispiele sind Isooctylalkohol, Isononylalkohol, Isostearylalkohol oder Isotridecylalkohol. Unter diesen ist Isononylalkohol besonders bevorzugt. Bei mehreren Methylgruppen beträgt deren Anzahl bevorzugt 2 bis 6, beliebig über den Alkylrest des Alkohols verteilt. Sofern es sich um Alkohole der Gruppe b2) mit einer C₂-C₁₈-Alkylgruppe als Verzweigung handelt, sind vorzugsweise keine weiteren Verzweigungen im Alkylrest des Alkohols vorhanden.
Weitere geeignete primäre monofunktionelle verzweigte Alkohole sind die dem Fachmann bekannten Guerbetalkohole, die bekanntlich durch Dimerisierung von Fettalkoholen zugänglich sind und sich strukturell dadurch auszeichnen, dass sie in α-Stellung zur endständigen CH₂OH-Gruppe einen längeren Alkylrest, vorzugsweise mit 2 bis 18 Kohlenstoffatomen, aufweisen. Geeignete Guerbetalkohole sind 2-Hexyldecanol, 2-Butyloctanol, 2-Octyldodecanol und 2-Hexyldecylpalmitat/stearat, 2-Ethylhexanol und 2-Propylheptanol. Bevorzugt ist 2- Ethylhexylalkohol.

Als Alkohole der Gruppe c), also difunktionelle Alkohole (mit 2 Hydroxylgruppen), können gesättigte oder ungesättigte Diole eingesetzt werden wie 1,5-Pentandiol, 1,8-Octandiol, 1,6-Hexandiol, 1,10-Decandiol, 1,12-Dodecandiol, 1,12-Octadecandiol oder die dem Fachmann bekannten Dimerdiole.

Die Poly-α-Olefine können in der erfindungsgemäßen kosmetischen Zusammensetzung in ungesättigter Form eingesetzt werden. Wegen der besseren Oxidationsbeständigkeit ist es jedoch bevorzugt, die Poly-α-Olefine im Anschluss an die dehydratisierende Polymerisation zu hydrieren und sie in der hydrierten (gehärteten) Form in den erfindungsgemäßen Zusammensetzungen zu verwenden.

Die Hydrierung ist in der bereits erwähnten Internationalen Anmeldung PCT/EP02/11392 beschrieben und kann In an sich bekannter Weise bei Temperaturen im Bereich von 150° bis 250°C, vorzugsweise 190° bis 210 °C, und Drücken von 20 bis 150 bar (Niederdruck-Verfahren) oder 150 bis 350 bar (Hochdruck-Verfahren) erfolgen. Als Katalysatoren eignen sich die aus dem Stand der Technik bekannten Hydrlerkatalysatoren wie Nickel oder die Edelmelallkatalysatoren, insbesondere auf Basis von Platin oder Palladium. Als besonders geeignet Edelmatallkatalysatoren haben sich Palladiumkatalysatoren erwiesen, insbesondere Palladium auf Kohle. Man kann den Katalysator In Form einer Suspension oder in fester Form zu den Poly-α-Olefinen in üblichen Mengen geben, die für das bevorzugte Palladium auf Kohle Im Bereich von 0,001 bis 5 Gew.% - berechnet als Palladium - liegt. Es ist jedoch auch möglich, den Katalysator auf einem festen Trägermaterial anzuordnen wie Aktivkohle, Graphit, Kleselgur, Kieselgel, Spinelle, Aluminiumoxid oder keramischen Materialien. Ebenso haben sich die Nickelkatalysatoren, beispielsweise suspendiertes Nickel wie Nysofact 101 I a (Fa. Engelhard) als geeignet erwiesen, welches vorzugsweise in Mengen von 0,01 bis 5 Gew.% - bezogen auf Nickel - eingesetzt wird.

Die beschriebenen Poly-α-Olefine sind, wie erwähnt, farblose bis leicht geibliche, praktisch geruchlose Verbindungen mit hohen Spreitlwerten, typischerweise größer als 1000 mm²/10 Minuten und vorzugsweise größer als 1600 mm²/10 Minuten (Definition nach Zeidler) liegen. Sie eignen sich daher ausgezeichnet zur Verwendung als Ölkörper in kosmetischen oder pharmazeutischen Zubereitungen. Wenn nachfolgend allgemein von Poly-α-Olefinen die Rede ist, sind damit sowohl die hydrierten als auch die nicht hydrierten Verbindungen gemeint.

Ein weiterer gegenstand der Erfindung betrifft die Verwendung von Poly-α-olefin, welches dadurch erhältlich ist, dass man wenigstens einen primären Alkohol in Gegenwart sauren Aluminoschichtsilikats einer dehydratisierenden Polymerisation bei einer Temperatur Im Bereich von 60 bis 340 °C unterwirft, wobei der primäre Alkohol ausgewählt ist aus der Gruppe
a) ungesättigter monofunktioneller Alkohole,
b) verzweigter monofunktioneller Alkohole und
c) difunktioneller Alkohole,
   und welches im Anschluss an die dehydratisierende Polymerisation hydriert wurde als Ölkörper in kosmetischen oder pharmazeutlschen Zubereitungen.

### Kosmetische Zubereitungen

Die erfindungsgemäße Verbindung erlaubt die Herstellung stabiler kosmetischer Emulsionen. Vorzugsweise handelt es sich hierbei um Formullerungen zur Körperpflege, z. B. In Form von Cremes, Milch, Lotionen, sprühbaren Emulsionen, Produkten zur Eliminierung des Körpergeruchs etc. Die erfindungsgemäßen Verbindung lässt sich auch in tensidhaltigen Formullerungen wie z. B. Schaum- und Duschbädern, Haarshampoos und Pflegespülungen einsetzen.

Die kosmetischen Mittel können in Form von Emulsionen oder Dispersionen vorliegen, die Wasser und Ölphase nebeneinander enthalten. Bevorzugte kosmetische Zusammensetzungen sind solche In Form einer W/O- oder O/W-Emulsion mit den üblichen, dem Fachmann geläuflgen, Konzentrationen an Ölen/Fetten/Wachsen, Emulgatoren, Wasser und den in der Kosmetik üblichen, weiteren Hilfs- und Zusatzstoffen.

Zweckmäßig enthält die erfindungsgemäße kosmetische Zusammensetzung 1 bis 50 Gew.-%, bevorzugt 5 bis 40 Gew.-% und insbesondere 5 bis 25 Gew.-%, Ölkörper, die zusammen mit z.B. öllöslichen Tensiden/Emulgatoren und öllöslichen Wirkstoffen Bestandteil der sogenannten Öl- oder Fettphase sind. Zu den Ölkörpern werden im Sinne der Erfindung Fettstoffe, Wachse, und flüssige Öle gerechnet, nicht aber Emulgatoren/Tenside. Die Poly-α-Olefine können als einziger Ölkörper oder aber in Kombination mit anderen Ölen/Fetten/Wachsen enthalten sein. Bevorzugt liegt der Anteil des wenigstens einen Poly-α-Olefins bezogen auf die Gesamtmenge der Ölkörper bei 0,1 bis 100 Gew.-% und bevorzugt bei 1 bis 50 Gew.-%. Mengen von 1 bis 20 Gew.-% und insbesondere 3 bis 20 Gew.-% sind besonders bevorzugt.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Die Mengen der jeweiligen Zusätze richten sich nach der beabsichtigten Verwendung. In einer weiteren bevorzugten Ausführungsform enthält die kosmetische Zusammensetzung 0,1 bis 20 Gew.-%, vorzugsweise 1 - 15 Gew.-% und insbesondere 1 - 10 Gew.-% einer oberflächenaktiven Substanz oder eines Gemisches aus oberflächenaktiven Substanzen.

### Oberflächenaktive Substanzen

Als oberflächenaktive Stoffe können anionische, nichtionische, kationische und/oder amphotere bzw. zwitterionische Tenside bzw. Emulgatoren oder ein beliebiges Gemisch dieser Tenside/Emulgatoren enthalten sein. In tensidhaltigen kosmetischen Zubereitungen, wie beispielsweise Duschgelen, Schaumbädern, Shampoos etc. ist vorzugsweise wenigstens ein anionisches Tensid enthalten, in Cremes und Lotionen für die Körperpflege sind vorzugsweise nicht-ionische Tenside/Emulgatoren enthalten.

Typische Beispiele für **anionische Tenside** sind Seifen, Alkylbenzolsulfonate, Alkansulfonate, Olefinsulfonäte, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Polyglycerinester, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel wie Cremes, Lotionen und Milch enthalten üblicherweise eine Reihe weiterer Ölkörper und Emollients, die dazu beitragen, die sensorischen Eigenschaften weiter zu optimieren. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleyllsostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol und Isopropanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylylcarbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z. B. Mineralöl, Vaseline, Petrolatum, Isohexadecane, Squalan, Squalen oder Dialkylcyclohexane in Betracht.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestem höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Verdickungsmittel

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Betone^{®} Gel VS-5PC (Rheox) erwiesen, bei dem es sich um eine Mischung aus Cyclopentasiloxan, Disteardimonium Hectorit und Propylencarbonat handelt, sowie ein Natriumpolyacrylat, das unter der Bezeichnung Cosmedia^{®} SP bekannt ist. Weiter in Frage kommen Elektrolyte wie Kochsalz und Ammoniumchlorid.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
➢ 3-Benzylidencampher bzw. 3-Benzylidennorcampher und dessen Derivate, z. B. 3-(4-Methylbenzyliden)campher
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester
➢ Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Octocrylene)
➢ Ester der Salizylsäure, vorzugsweise Salicylsäure-2-ethylhexylester, Salicylsäure-4-iso-propylbenzylester, Salicylsäurehomomenthylester
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
➢ Ester der Benzalmalonsäure, vorzugsweise 4-Methoxybenzmalonsäuredi-2-ethylhexyl-ester
➢ Triazinderivate, wie z. B. 2,4,6-Trianilino-(p-carbo-2'-ethyl-1'-hexyloxy)-1,3,5-triazin und Octyl-Triazon oder Dioctyl Butamido Triazone (Uvasorb® HEB)
➢ Propan-1,3-dione, wie z. B. 1-(4-tert.Butylphenyl)-3-(4'methoxyphenyl)propan-1,3-dion ➢ Ketotricyclo(5.2.1.0)decan-Derivate

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze sowie 2,2-(1,4-Phenylene)bis-1H-benzimidazole-4,6-disulfonsäure und deren Salze, insbesondere das Natrium-Salz,
➢ Sulfonsäurederivate von Benzophenonen, vorzugsweise 2-Hydroxy-4-methoxybenzo-phenon-5-sulfonsäure und ihre Salze
➢ Sulfonsäurederivate des 3-Benzylidencamphers, wie z. B. 4-(2-Oxo-3-bomylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Desodorierende Wirkstoffe

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen.

### Anitranspirante Wirkstoffe

Antitranspirant-Wirkstoffe reduzieren durch Beeinflussung der Aktivität der ekkrinen Schweißdrüsen die Schweißbildung, und wirken somit Achselnässe und Körpergeruch entgegen.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2, Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2,4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwefelteer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzem, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

### Beispiele

In den nachfolgenden Beispielen bedeuten:

| | |
|---|---|
| SZ | Säurezahl |
| IZ | Iodzahl |
| OHZ | Hydroxylzahl. |

### Beispiel 1

2700 g Isononylalkohol wurden in Gegenwart von 5 Gew.% des Katalysators K5 unter Stickstoffatmosphäre erhitzt bis eine Wasserabscheidung beobachtet wurde. Bei dieser Temperatur wurde die Reaktion gehalten und solange durchgeführt bis keine weitere Abspaltung von Wasser erfolgte. Der noch heiße Reaktionsansatz wurde vom Katalysator durch Filtration entfernt und lieferte ein blankes, farbloses Produkt.
Analytik: SZ= 0,1; IZ=167; OHZ= 0,7.

### Beispiel 2

1200 g 2-Ethylhexanol wurden in Gegenwart von 5 Gew.% des Katalysators K5 unter Stickstoffatmosphäre erhitzt bis eine Wasserabscheidung beobachtet wurde. Bei dieser Temperatur wurde die Reaktionsmischung solange gehalten, bis keine weitere Abspaltung des Wassers erfolgte. Der noch heiße Reaktionsansatz wurde vom Katalysator durch Filtration entfernt. Nach Abdestillation der Leichtsieder erhielt man blankes, farbloses Produkt.
Analytik: SZ = 0,2; IZ = 217; OHZ = 0,4.

### Beispiel 3

1030 g Poly-α-Olefin auf Basis von Isononylalkohol (erhalten aus Beispiel 1) und 0.05% Gew.% Palladium auf Kohle wurde 5 Stunden bei 200 °C mit 100 bar Wasserstoff behandelt. Der Katalysator wurde abfiltriert und das Produkt desodoriert.
Analytik: OHZ =0,9; IZ:1,6; SZ: 0,1.

### Beispiel 4

560 g Poly-α-Olefin auf Basis von 2-Ethylhexanol (erhalten aus Beispiel 2) und 0.05% Gew.% Palladium auf Kohle wurde 5 Stunden bei 200 °C mit 100 bar Wasserstoff behandelt. Der Katalysator wurde abfiltriert und das Produkt desodoriert.
Analytik: OHZ=0,1; IZ:1,1; SZ: 0,1.

Kosmetische Zusammensetzungen

### Beispiel 5

Mit dem Poly-α-Olefin gemäß Beispiel 3 als Ölkomponente wurde folgende O/W-Emulsion hergestellt:

| | | |
|---|---|---|
| Eumulgin^{®} B2 | 2 Gew.% | Gew.% |
| Lanette^{®} O | 5 Gew.-% | Gew.% |
| Ölkomponente | 16 Gew.-% | Gew.% |
| Glycerin | 3 Gew.-% | Gew.% |
| Wasser | 73,85 Gew.-% | Gew.% |
| Formalin (37 %ig) | 0,15 Gew.-% | Gew.% |

### Beispiel 6

Mit dem Poly-α-Olefin gemäß Beispiel 3 als Ölkomponente wurde folgende O/W-Emulsion hergestellt:

| | | |
|---|---|---|
| Eumulgin^{®} VL 75 | 4,5 | Gew.% |
| Ölkomponente | 16 | Gew.% |
| Carbopol^{®} | 0,3 | Gew.% |
| KOH (20 %ig) | 0,7 | Gew.% |
| Glycerin | 3 | Gew.% |
| Wasser | 75,35 | Gew.% |
| Formalin (37 %ig) | 0,15 | Gew.% |

### Beispiel 7

Mit dem Poly-α-Olefin gemäß Beispiel 3 als Ölkomponente wurde folgende W/O-Emulsion hergestellt:

| | | |
|---|---|---|
| Dehymuls^{®} PGPH | 5 | Gew.% |
| Ölkomponente | 20 | Gew.% |
| Glycerin | 5 | Gew.% |
| Mg-Sulfat · 7 H₂O | 1 | Gew.% |
| Wasser | 68,85 | Gew.% |
| Formalin (37 %ig) | 0,15 | Gew.% |

Das Isononyl-Oligomer-Polyalphaolefin hatte eine konstante Viskosität (ca. 5000 mPas) über die Lagerzeit von 12 Wochen bei Raumtemperatur. Sowohl bei - 5 °C als auch bei 40, 45 und 50 °C waren die Emulsionen mit Carbopol^{®} über 12 Wochen stabil.
Die anschließende Tabelle 1 zeigt die Untersuchungen zu Viskosität und Lagerstabilität der Emulsion gemäß Beispiel 6 im Vergleich zu Emulsionen, die als Ölkomponente Nexbase^{®} 2006 FG oder dünnflüssiges Paraffinöl enthielten. Im Gegensatz zu der erfindungsgemäßen Emulsion waren die Vergleichs-Emulsionen unter diesen Bedingungen nach etwa 1 Woche gebrochen.

**Tabelle 1**

| | Isononyl-Oligomer hydriert | Paraffinöl dünnfl. | Nexbase^{®} 2006FG |
|---|---|---|---|
| Eumulgin^{®} VL 75 | 4,50 | 4,50 | 4,50 |
| Isononyt-oligom. hydriert | 16,00 | - - | - - |
| Paraffinöl dünnfl. | - - | 16,00 | - - |
| Nexbase^{®} 2006FG | - - | - - | 16,00 |
| Carbopol^{®} | 0,30 | 0,30 | 0,30 |
| KOH 20 %ig | 0,70 | 0,70 | 0,70 |
| Glycerin 86%ig | 3,00 | 3,00 | 3,00 |
| Wasser entsalzt | 75,35 | 75,35 | 75,35 |
| Formalin 37 %ig | 0,15 | 0,15 | 0,15 |

| **Viskosität in mPas** | | | |
|---|---|---|---|
| Tag 1 | 4000 | 4800 | 4800 |
| 1 Woche | 4000 | 6400 | 6000 |
| 4 Wochen | 4800 | -- | -- |
| 8 Wochen | 5200 | -- | -- |
| 12 Wochen | 4800 | - - | -- |

| **Stabilitäten *** | | | |
|---|---|---|---|
| 1 Woche RT/-5°C /40°C/45°C/50°C | 1/1/1/1/1 | 1/1/1/5/5 | 1/1/1/1/1 |
| 4 Wochen RT/-5°C /40°C/45°C/50°C | 1/1/1/1/1 | 5/1/5/-/- | 5/5/5/5/5 |
| 8 Wochen RT/-5°C /40°C/45°C/50°C | 1/1/1/1/1 | -/1/-/-/ | -/-/-/-/- |
| 12 Wochen RT/-5°C /40°C/45°C/50°C | 1/1/1/1/1 | | -/-/-/-/- |

| | | | |
|---|---|---|---|
| * In Tabelle 1 bezeichnet "1" stabile Emulsionen, "5" nicht stabile Emulsionen und "-" solche Emulsionen, die offensichtlich instabil, d.h. entmischt, waren. RT = Raumtemperatur | | | |

Im Weiteren sind Beispielrezepturen aufgeführt, welche die vielfältigen Einsatzmöglichkeiten der erfindungsgemäßen kosmetischen Zusammensetzungen demonstrieren. Mengenangaben beziehen sich auf Gew.-% der handelsüblichen Substanzen in der Gesamtzusammensetzung.

**Tabelle 2: O/W-Sonnenschutzemulsionen**

| | | | | | | | | | | | |
|---|---|---|---|---|---|---|---|---|---|---|---|
| **Komponente** | **1** | **2** | **3** | **4** | **5** | **6** | **7** | **8** | **9** | **10** | **11** |
| **L = Lotion, C= Creme,** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulgin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®}O | 1 | 6 | | | 5 | 2 | | 2 | | | |
| Antaron V 216 | | | 1 | | 2 | 2 | | | | 1 | |
| Emery^{®} 1780 | | | | | 0.5 | 0.5 | | | | | |
| Lanolin, wasserfrei USP | | | | | | | 5 | | | | |
| Poly-α-Olefin (Bsp.4) | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.7 | | | | | 1 | 1 | | |
| Keltrol^{®} T | | | 0.2 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH Wasser | q.s. ad 100 | | | | | | | | | | |

**Tabelle 3: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Myrj^{®} 51 | | | | | | | | | | | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{®} O | | | | 2 | | | | | 1 | 1 | |
| Antaron V 220 | 1 | | | 2 | | | 0.5 | | | 2 | 0.5 |
| Poly-α-Olefin (Bsp. 4) | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 4: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Microkristallines Wachs | | | 5 | | | 2 | | | | | 5 |
| Bienenwachs | 1 | | | 1 | | | | 5 | | 7 | |
| Tego^{®} Care CG | | | | | 1 | | | | | | 5 |
| Prisorine^{®} 3505 | 1 | | 1 | 1 | | 1 | 1 | | | | 1 |
| Emery^{®} 1780 | | | 5 | | | | | | | 4 | |
| Wollwachsalkohol, wasserfrei, | | | | | | | | | | | 1 |
| Antaron V 216 | 2 | | | | | | | | | | |
| Poly-α-Olefin (Bsp.4) | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G | | | 3 | | | | | 8 | | | |
| Cetiol^{®} PGL | | 11 | | | | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 5: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L= Lotion; C= Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-O18 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Mikrokristallines Wachs | | | | | 4 | | 1 | | | 4 | |
| Bienenwachs | | 4 | | 2 | | | 1 | | 2 | | 1 |
| Tego^{®} Care CG | | | | | | | | | | | |
| Isostearinsäure | 1 | 1 | | | | | 1 | 1 | | 1 | 1 |
| Emery^{®} 1780 | | 7 | 3 | | | | | | | | |
| Wollwachsalkohol, wasserfrei, | | | | | | | | | | | |
| Antaron V 220 | | 0.5 | 2 | 1 | 1 | | | | | | |
| Poly-α-Olefin (Bsp. 4) | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G | | | | 2 | | | | | 5 | | |
| Cetiol^{®} PGL | | | | | | | | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 6: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGl | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| Poly-α-Olefin (Bsp. 4) | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 7: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-018 | | 1 | | 1 | | | | | | | |
| Lameform^{®} TGI | | | | | 4 | | | 1 | | 3 | |
| Abil^{®} EM 90 | | | | 3 | | | | | | 2 | |
| Isolan^{®} PDI | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Mikrokristallines Wachs | | | | | 4 | | 1 | | | 4 | |
| Bienenwachs | | 4 | | 2 | | 2 | 1 | 1 | 2 | | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Poly-α-Olefin (Bsp.4) | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning^{®} DC 244 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G 16 | 1 | | | | | | | | | | |
| Eutanol^{®} G | | | | 2 | | | | | 5 | | |
| Cetiol^{®} PGL | | | 10 | | | | | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 8: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C= Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{®} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 5 | | | | |
| Cetiol^{®} SB 45 | | | 1.5 | | | | 2 | | | | |
| Poly-α-Olefin (Bsp. 4) | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | | | | | | 4 | 3 | |
| Dow Corning DC^{®} 245 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | 0.3 | 0.3 | 0.2 | 0.2 | 0.2 | | | 0.1 | 0.3 | 0.2 |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., pH 6,5 - 7,5 | | | | | | | | | | |

**Tabelle 9: O/W-Pflegeemulsionen**

| **Komponente** | **78** | **79** | **80** | **81** | **82** | **83** | **84** | **85** | **86** | **87** | **88** |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{®} O | 2 | | | 2 | | 3 | 1 | | 1 | 1 | 6 |
| Novata^{®} AB | | | | | | | | | | | |
| Emery^{®} 1780 | | | | | | | | | | | |
| Lanolin, wasserfrei, USP | | | | | | 4 | | | | | |
| Cetiol^{®} SB 45 | | | | | | | 2 | | | | |
| Poly-α-Olefin (Bsp. 4) | 3 | 4 | 2 | 1 | 10 | 2 | 2 | 6 | 3 | 12 | 1 |
| Cegesoft^{®} C 17 | 4 | | | | | | | | | | |
| Myritol^{®} PC 6 | | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Tabelle 10: Sprayformulierungen**

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| Antaron V 220 | | | | | | 1 | 1 | 1 | | 1 | 1 |
| Poly-α-Olefin (Bsp. 4) | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 5 | | | 2 | | | | | |
| Dow Corning DC^{®} 244 | | 4 | 4 | 5 | | | | | | | |
| Cetiol^{®} 868 | 3 | | | | | | | | | | |
| Cetiol^{®} J 600 , | | | | 2 | 2 | | | | | | |
| Mineralöl | | | 2 | | | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusoiex^{®} T 2000 | | | | | | | | | | 2 | 2 |
| Veegum^{®} Ultra | | | | | | | | | | | 1.5 |
| Laponite^{®}XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.s./ q.s | | | | | | | | | | |

### ANHANG

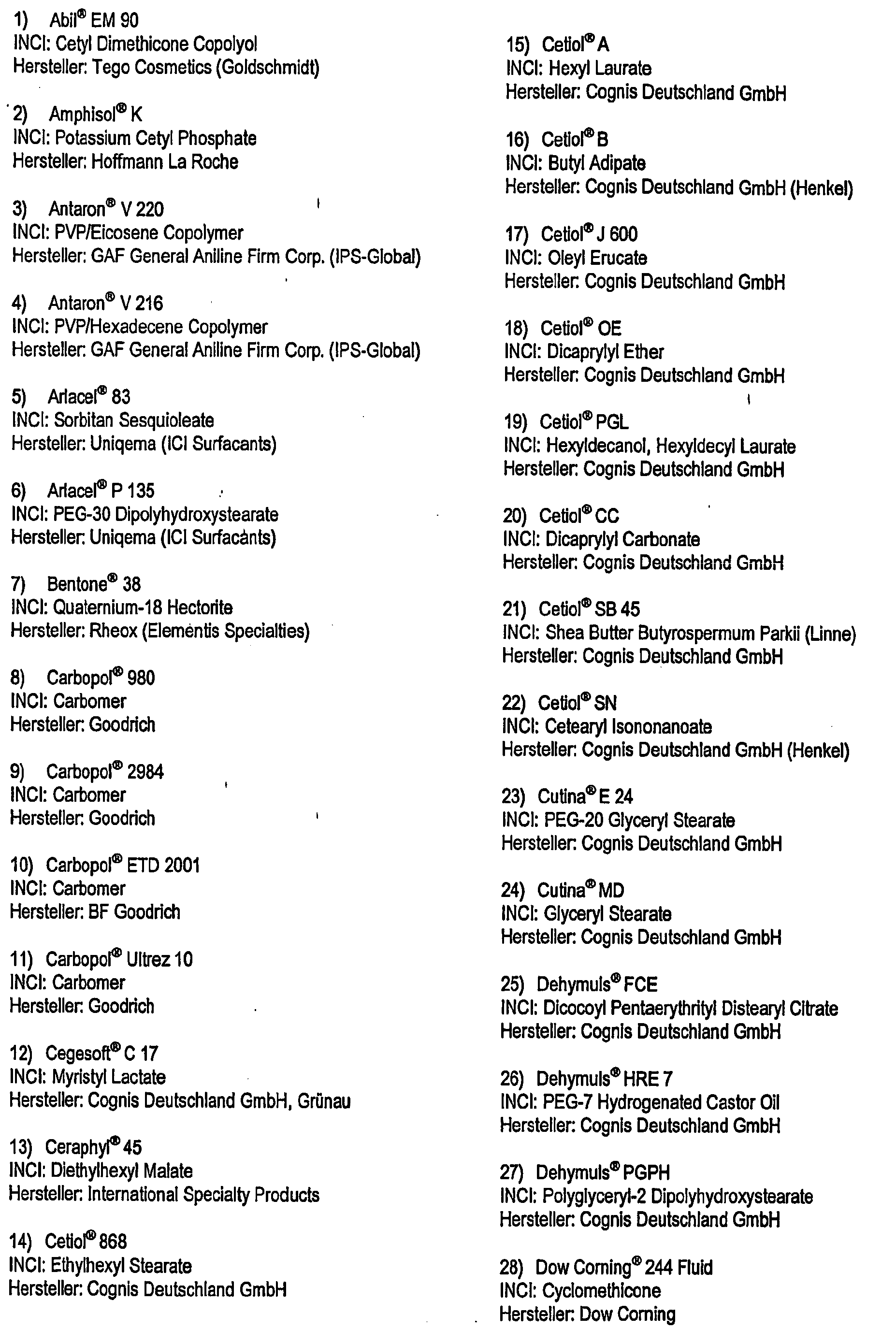

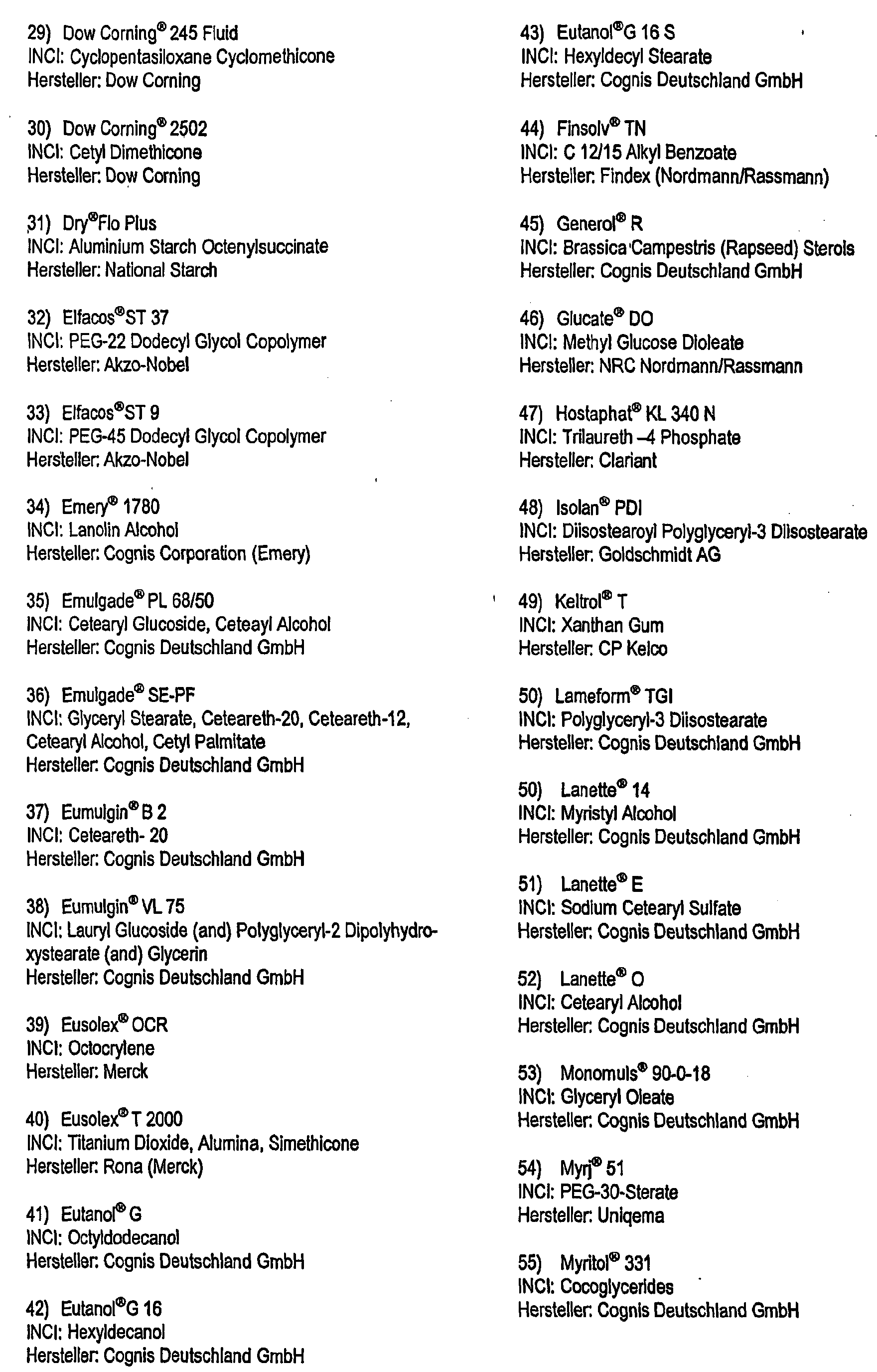

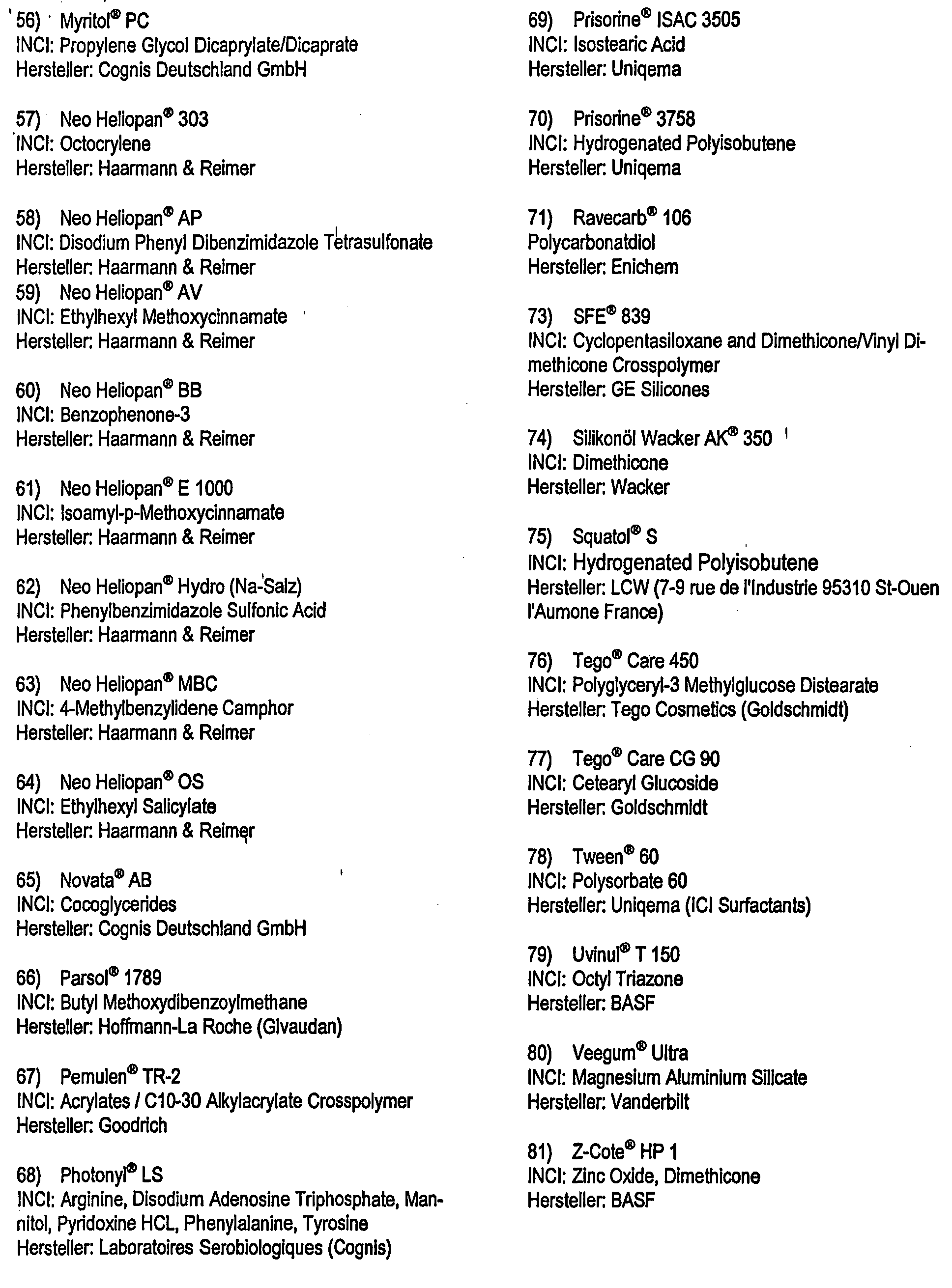

## Patentansprüche

1. Kosmetische Zusammensetzung in Form von Emulsionen oder Dispersionen, die Wasser und Ölphase nebeneinander enthalten, **dadurch gekennzeichnet, dass** sie wenigstens ein Poly-α-Olefin enthält, welches dadurch erhältlich ist, dass man wenigstens einen primären Alkohol in Gegenwart sauren Aluminoschichtsilikats einer dehydratisierenden Polymerisation bei einer Temperatur im Bereich von 60 bis 340 °C unterwirft, wobei der primäre Alkohol ausgewählt ist aus der Gruppe
a) ungesättigter monofunktioneller Alkohole,
b) verzweigter monofunktioneller Alkohole und
c) difunktioneller Alkohole,

2. Kosmetische Zusammensetzung nach Anspruch 1, **dadurch gekennzeichnet, dass** der primäre Alkohol 6 bis 72 Kohlenstoffatome und insbesondere 6 bis 24 Kohlenstoffatome aufweist.

3. Kosmetische Zusammensetzung nach Anspruch 1 oder 2, **dadurch gekennzeichnet, dass** als Alkohol der Gruppe b) ein Alkohol eingeseizt wird, der ausgewählt ist aus der Gruppe der verzwelgten Alkohole mit
b1) mindestens einer Methylgruppe und insbesondere 1 bis 6 Mothylverzweigungen im Alkylrest
b2) einer C₂-C₁₈-Verzweigung im Alkylrest und
b3) einer G₂-C₁₈-Verzwelgung in α-Stellung zur endständigen CH₂OH-Gruppe.

4. Kosmetische Zusammensetzung nach Anspruch 3, **dadurch gekennzeichnet, dass** als Alkohol ein Guerbetalkohol, insbesondere 2-Ethylhexylalkohol, oder isononylalkohol eingesetzt wird.

5. Kosmetische Zusammensetzung nach Anspruch 1 oder 2, **dadurch gekennzeichnet, dass** als Alkohol der Gruppe a) ein linearer Alkohol eingesetzt wird.

6. Kosmetische Zusammensetzung nach einem der Ansprüche 1 bis 4, **dadurch gekennzeichnet, dass** der Alkohol ausgewählt ist aus der Gruppe b) oder c) und das Poly-α-Olefin im Anschluss an die dehydratlslerende Polymerisation hydriert wird.

7. Kosmetische Zusammensetzung nach einem der Ansprüche 1 bis 6, **dadurch gekennzeichnet, dass** sie In Form einer W/O- oder O/W-Emulsion vorliegt.

8. Kosmetische Zusammensetzung nach einem der Ansprüche 1 bis 7, **dadurch gekennzeichnet, dass** sie 1 bis 50 Gew.-%, bevorzugt 5 bis 40 Gew.-% und insbesondere 5 bis 25 Gew.-%, Ölkörper enthält.

9. Kosmetische Zusammensetzung nach einem der Ansprüche 1 bis 8, **dadurch gekennzeichnet, dass** der Anteil des wenigstens einen Poly-α-Olefins bezogen auf die Gesamtmenge der Ölkörper 0,1 bis 100 Gew.-%, bevorzugt 1 bis 50 Gel.-%, besonders bevorzugt 1 bis 20 Gew.-% und Insbesondere 3 bis 20 Gew.-%, beträgt.

10. Kosmetische Zusammensetzung nach einem der Ansprüche 1 bis 9, **dadurch gekennzeichnet, dass** sie 0,1 bis 20 Gew,-% vorzugsweise 1-15 Gew.-% und insbesondere 1-10 Gew.-% einer oberflächenaktiven Substanz oder eines Gemisches aus oberflächenaktiven Substanzen enthält,

11. Verwendung von Poly-α-Olefin, welches **dadurch** erhältlich ist, dass man wenigstens einen primären Alkohol in Gegenwart sauren Aluminoschichtsilikats einer dehydratlslerenden Polymerisation bei einer Temperatur im Bereich von 60 bis 340 °C unterwirft, wobei der primäre Alkohol ausgewählt Ist aus der Gruppe
a) ungesättigter monofunktioneller Alkohole,
b) verzweigter monofunktioneller Alkohole und
c) difunktioneller Alkohole,
und welches im Anschluss an die dehydratisierende Polymerisation hydriert wurde als Ölkörper In kosmetischen oder pharmazeutischen Zubereitungen.

## Claims

1. Cosmetic composition in the form of emulsions, which contain water and the oil phase alongside one another, **characterized in that** it contains at least one poly-α-olefin obtainable by subjecting at least one primary alcohol to dehydrating polymerization at 60 to 340°C in the presence of acidic alumino layer silicate, the primary alcohol being selected from the group consisting of
a) unsaturated monofunctional alcohols,
b) branched monofunctional alcohols and
c) difunctional alcohols.

2. Cosmetic composition as claimed in claim 1, **characterized in that** the primary alcohol contains 6 to 72 carbon atoms and more particularly 6 to 24 carbon atoms.

3. Cosmetic composition as claimed in claim 1 or 2, **characterized in that** the group b) alcohol used is an alcohol selected from the group of branched alcohols having
b1) at least one methyl group and, more particularly, 1 to 6 methyl branches in the alkyl chain,
b2) a C₂₋₁₈ branch in the alkyl chain and
b3) a C₂₋₁₈ branch in the α-position to the terminal CH₂OH group.

4. Cosmetic composition as claimed in claim 3, **characterized in that** a Guerbet alcohol, more particularly 2-ethylhexyl alcohol, or isononyl alcohol is used as the alcohol.

5. Cosmetic composition as claimed in claim 1 or 2, **characterized in that** a linear alcohol is used as the group a) alcohol.

6. Cosmetic composition as claimed in any of claims 1 to 4, **characterized in that** the alcohol is selected from group b) or c) and the poly-α-olefin is hydrogenated after the dehydrating polymerization.

7. Cosmetic composition as claimed in any of claims 1 to 6, **characterized in that** it is present in the form of a w/o or o/w emulsion.

8. Cosmetic composition as claimed in any of claims 1 to 7, **characterized in that** it contains 1 to 50% by weight, preferably 5 to 40% by weight and more particularly 5 to 25% by weight of oil components.

9. Cosmetic composition as claimed in any of claims 1 to 8, **characterized in that** the percentage content of the at least one poly-α-olefin, based on the total quantity of oil components, is 0.1 to 100% by weight, preferably 1 to 50% by weight, more preferably 1 to 20% by weight and most preferably 3 to 20% by weight.

10. A cosmetic composition as claimed in any of claims 1 to 9, **characterized in that** it contains 0.1 to 20% by weight, preferably 1 to 15% by weight and more particularly 1 to 10% by weight of a surface-active substance or a mixture of surface-active substances.

11. The use of poly-α-olefin obtainable by subjecting at least one primary alcohol to dehydrating polymerization at 60 to 340°C in the presence of acidic alumino layer silicate, the primary alcohol being selected from the group consisting of
a) unsaturated monofunctional alcohols,
b) branched monofunctional alcohols and
c) difunctional alcohols,
and which were hydrogenated after the dehydrating polymerization, as oil components in cosmetic or pharmaceutical preparations.

## Revendications

1. Composition cosmétique sous forme d'émulsions ou de dispersions qui contiennent une phase aqueuse et une phase huileuse coexistantes, **caractérisée en ce qu'**elle contient au moins une poly-α-oléfine qui peut être obtenue par le fait qu'on soumet au moins un alcool primaire, en présence d'aluminosilicates lamellaires acides, à une polymérisation déshydratante à une température dans la plage de 60 à 340 °C, l'alcool primaire étant choisi dans le groupe
a) des alcools monofonctionnels insaturés,
b) des alcools monofonctionnels ramifiés et
c) des alcools bifonctionnels.

2. Composition cosmétique selon la revendication 1, **caractérisée en ce que** l'alcool primaire a de 6 à 72 atomes de carbone et en particulier de 6 à 24 atomes de carbone.

3. Composition cosmétique selon la revendication 1 ou 2, **caractérisée en ce qu'**on utilise comme alcool du groupe b) un alcool qui est choisi dans le groupe des alcools ramifiés comportant
b1) au moins un groupe méthyle et en particulier 1 à 6 ramifications méthyle dans le radical alkyle et
b2) une ramification en C₂-C₁₈ dans le radical alkyle et
b3) une ramification en C₂-C₁₈ en position α par rapport au groupe CH₂OH en bout de chaîne.

4. Composition cosmétique selon la revendication 3, **caractérisée en ce qu'**on utilise comme alcool un alcool de Guerbet, en particulier l'alcool 2-éthylhexylique, ou l'alcool isononylique.

5. Composition cosmétique selon la revendication 1 ou 2, **caractérisée en ce qu'**on utilise comme alcool du groupe a) un alcool linéaire.

6. Composition cosmétique selon l'une quelconque des revendications 1 à 4, **caractérisée en ce que** l'alcool est choisi dans le groupe b) ou c) et la poly-α-oléfine est hydrogénée à la suite de la polymérisation déshydratante.

7. Composition cosmétique selon l'une quelconque des revendications 1 à 6, **caractérisée_en ce qu**'elle se trouve sous forme d'une émulsion E/H ou H/E.

8. Composition cosmétique selon l'une quelconque des revendications 1 à 7, **caractérisée en ce qu'**elle contient de 1 à 50 % en poids, de préférence de 5 à 40 % en poids et en particulier de 5 à 25 % en poids de corps huileux.

9. Composition cosmétique selon l'une quelconque des revendications 1 à 8, **caractérisée en ce que** la proportion de ladite au moins une poly-a-oléfine, par rapport à la quantité totale des corps huileux, va de 0,1 à 100 % en poids, de préférence de 1 à 50 % en poids, de façon particulièrement préférée de 1 à 20 % en poids et en particulier de 3 à 20 % en poids.

10. Composition cosmétique selon l'une quelconque des revendications 1 à 9, **caractérisée en ce qu'**elle contient de 0,1 à 20 % en poids, de préférence de 1 à 15 % en poids et en particulier de 1 à 10 % en poids d'une substance tensioactive ou d'un mélange de substances tensioactives.

11. Utilisation d'une poly-α-oléfine qui peut être obtenue par le fait qu'on soumet au moins un alcool primaire, en présence d'aluminosilicates lamellaires acides, à une polymérisation déshydratante à une température dans la plage de 60 à 340 °C, l'alcool primaire étant choisi dans le groupe
a) des alcools monofonctionnels insaturés,
b) des alcools monofonctionnels ramifiés et
c) des alcools bifonctionnels,
et qui a été hydrogénée à la suite de la polymérisation déshydratante, en tant que corps huileux dans des préparations cosmétiques ou pharmaceutiques.
